**Europäisches Patentamt**

**European Patent Office**

**Office européen des brevets**

(11) Veröffentlichungsnummer: **0 188 719**
**B1**

(12) **EUROPÄISCHE PATENTSCHRIFT**

(45) Veröffentlichungstag der Patentschrift: **04.07.90**

(51) Int. Cl.⁵: **C 07 C 47/42, C 07 C 45/69**

(21) Anmeldenummer: **85115516.8**

(22) Anmeldetag: **06.12.85**

(54) **Verfahren zur Herstellung von 3-Cyclohexen-1-carboxaldehyd.**

(30) Priorität: **19.01.85 DE 3501665**

(43) Veröffentlichungstag der Anmeldung:
**30.07.86 Patentblatt 86/31**

(45) Bekanntmachung des Hinweises auf die
Patenterteilung:
**04.07.90 Patentblatt 90/27**

(84) Benannte Vertragsstaaten:
**AT CH DE FR GB IT LI NL SE**

(56) Entgegenhaltungen:
**GB-A- 309 911**

**CHEMICAL ABSTRACTS, Band 92, 1980, Seite
547, Zusammenfassung Nr. 146312z, Columbus,
Ohio, US; J. MARKOS: "Modeling of a chemical
reactor for consecutive reactions"**

(73) Patentinhaber: **Degussa Aktiengesellschaft
Weissfrauenstrasse 9
D-6000 Frankfurt am Main 1 (DE)**

(72) Erfinder: **Prescher, Günter, Dr. Dipl.-Chem.
Liesingstrasse 2
D-6450 Hanau 9 (DE)**
Erfinder: **Grund, Andreas, Dr. Dipl.-Chem.
Rilkeweg 15
D-6100 Darmstadt (DE)**
Erfinder: **Petsch, Heinrich, Ing. grad.
Tulpenstrasse 23
D-6450 Hanau 8 (DE)**
Erfinder: **Böhme, Georg
Nordring 49
D-6458 Rodenbach (DE)**

Courier Press, Leamington Spa, England.

## Beschreibung

Die vorliegende Erfindung betrifft ein Verfahren zur Herstellung von 3-Cyclohexen-1-carboxaldehyd.

Es ist seit langem bekannt, beispielsweise von O. Diels und K. Alder (Liebigs Ann. Chem. *460*, 98 (1928)) oder aus den Patentschriften GB-PS 1,024,012 und GB-PS 309,911, daß sich Acrolein mit Butadien im Bombenrohr oder im Autoklaven unter diskontinuierlicher Arbeitsweise zu 3-Cyclohexen-1-carboxaldehyd umsetzen läßt. Die nach diesen bekannten Verfahren erzielten Umsätze und Ausbeuten sind nicht immer befriedigend, da der als Ausgangsverbindung verwendete α,ß-ungesättigte Aldehyd sowie das konjugierte Olefin leicht bei höherer Temperatur polymerisieren. Zum einen verläuft die diskutierte Reaktion sehr exotherm, zum anderen muß aber, um eine genügend hohe Reaktionsgeschwindigkeit zu erzielen, eine Reaktionstemperatur von im allgemeinen über 90°C herrschen. Da bei diesen Temperaturen bereits die Polymerisation der Ausgangsstoffe begünstigt ist, muß bei diskontinuierlicher Arbeitsweise äußerst vorsichtig aufgeheizt werden. Dies gilt vor allem dann, wenn erhebliche, äquimolare Mengen der Reaktionspartner miteinander zur Umsetzung gebracht werden, da die Gefahr einer örtlichen starken Überhitzung zu einer äußerst heftigen, nicht mehr zu beherrschenden Reaktion führen kann.

Man hat daher vorgeschlagen, die Umsetzung in inerten Lösungsmitteln oder bei niedrigen Temperaturen in z.B. Benzol als Solvens in Gegenwart aluminiumorganischer Verbindungen als Katalysatoren durchzuführen (US-PS 3 067233; GB-PS 835 840).

Die bei diesen Verfahren erzielten Ausbeuten an 3-Cyclohexen-1-carboxaldehyd sind jedoch im allgemeinen mäßig; außerdem erfordert der Einsatz von metallorganischen, äußerst leicht entzündlichen Katalysatoren völlig wasserfreie Ausgangsstoffe und Lösungsmittel, was neben der Rückgewinnung des Lösungsmittels einen erheblichen Aufwand im technischen Maßstab hervorruft.

Ein kontinuierliches Verfahren zur Herstellung von 3-Cyclohexen-1-carboxaldehyd aus Butadien und überschüssigem Acrolein in einem einzigen Reaktionsgefäß beschreibt die GB-PS 689 568. In diesem Prozeß wird als Einsatzstoff ein C4-Crackschnitt von nur 60% Butadiengehalt verwendet; das erforderliche Reaktorvolumen wird daher unverhältnismäßig groß, ferner fallen 8% Rückstand an. Desweiteren müssen die im Roh-Butadien enthaltenen sonstigen Bestandteile sowie erhebliche Mengen überschüssigen Acroleins abgetrennt und recycliert bzw. entsorgt werden.

Daher kann auch der vorgenannte kontinuierliche Prozeß für eine wirtschaftliche Herstellung von 3-Cyclohexen-1-carboxaldehyd in größerem Maßstab nicht herangezogen werden.

Gegenstand der Erfindung ist ein Verfahren zur Herstellung von 3-Cyclohexen-1-carboxaldehyd durch Diels-Alder-Reaktion von Butadien mit Acrolein bei erhöhter Temperatur und erhöhtem Druck, das dadurch gekennzeichnet ist, daß man die Umsetzung kontinuierlich in der Weise durchführt, daß zunächst in einem Umlaufreaktor, dem Acrolein und Butadien kontinuierlich zugeführt werden, bei einer Temperatur von 80—160°C, vorzugsweise 100—130°C, gearbeitet wird, wobei man einen Teil des Reaktionsgemisches im Kreis führt, und das Gewichtsverhältnis der pro Zeiteinheit zugeführten Ausgangsstoffe zu dem pro Zeiteinheit umgewälzten Reaktionsgut 1:5 bis 1:60 beträgt und man anschließend das den Umlaufreaktor verlassende Reaktionsgemisch, das mindestens 50 Gewichtsprozent 3-Cyclohexen-1-carboxaldehyd enthält, einem zweiten Umlaufreaktor zuführt, in dem ebenfalls ein Teil des Reaktionsgemisches im Kreis geführt wird, wobei das Gemischverhältnis des pro Zeiteinheit vom ersten Umlaufreaktor zugeführten Reaktionsgemisches zu der pro Zeiteinheit umgewälzten Reaktionslösung ebenfalls 1:5 bis 1:60 beträgt und in dem die Reaktion bei einer Temperatur zwischen 100°C und 200°C, vorzugsweise 110°C bis 150°C durchgeführt wird und die den zweiten Umlaufreaktor verlassende Reaktionslösung, die einen Gehalt von mindestens 75% 3-Cyclohexen-1-carboxaldehyd aufweist, einem Nachreaktor zuführt, in dem kein Umlauf des Reaktionsgemisches stattfindet und die Temperatur bei 100°C—240°C, vorzugsweise 140 bis 170°C liegt und man dann das erhaltene Reaktionsgemisch fraktioniert destilliert.

Die Erfindung ergibt somit eine vorteilhafte Lösung des Problems, da für einen wesentlichen Teil der Reaktion eine starke Rückvermischung mit bereits gebildetem Produkt erfolgt, was einen durch die Menge an zugeführten Ausgangsstoffen genau einstellbaren Verdünnungseffekt bewirkt. Zur Erzielung eines vollständigen Umsatzes wird dann in einem Nachreaktor ohne Umlauf, dem ein zweiter Umlaufreaktor vorgeschaltet sein kann, die Reaktion zu Ende geführt.

Nach dem erfindungsgemäßen Verfahren werden bei Umsätzen bis ca. 98% Ausbeuten von über 90% an 3-Cyclohexen-1-carboxaldehyd erzielt, wobei der Anteil an polymeren Stoffen oder hochsiedenden Produkten 1,5—3%, bezogen auf die eingesetzten Stoffe, in der Regel nicht überschreitet.

Geeignet für die Umsetzung ist Acrolein technischer Qualität, das ca. 3% Wassergehalt aufweist; es kann aber ebensogut wasserfreies Acrolein oder solches mit höheren Wassergehalten verwendet werden.

Das Molverhältnis von Acrolein und Butadien beträgt im allgemeinen 0,5:1 bis 2:1, vorzugsweise 0,9:1 bis 1,1:1.

Im allgemeinen wendet man in den vorteilhaft als Umlaufreaktoren eingesetzten Schlaufenreaktoren Reaktionstemperaturen von 80—200°C, vorzugsweise 100—150°C an; im Nachreaktor kann die Reaktionstemperatur 100—240°C, vorzugsweise 140—170°C betragen. Die Umsetzung wird im allgemeinen bei dem sich in den Reaktoren als Summe der partialdrücke der Reaktionsteilnehmer resultierenden Druck durchgeführt, welcher aufgrund der hohen Rückvermischung mit bereits gebildetem produkt sehr viel niedriger als bei vergleichbaren kontinuierlichen Verfahren liegt und dadurch apparativ zu erheblichen Vorteilen führt. Es ist jedoch auch möglich, höheren Druck, beispielsweise bis zu 200 bar anzuwenden.

Üblicherweise ist Acrolein gegen Polymerisation stabilisiert, so daß die Reaktion in Gegenwart des Polymerisationsinhibitors abläuft. Als Polymerisationsinhibitoren sind gängige Inhibitoren wie z.B. Hydrochinon verwendbar. Deren Konzentration beträgt, bezogen auf das Acrolein 0,05—1, vorzugsweise 0,1 bis 0,5 Gewichtsprozent.

Zur Durchführung des erfindungsgemäßen kontinuierlichen Verfahrens eignet sich z.B. ein bzw. zwei hintereinandergeschaltete Schlaufenreaktoren, aus denen ein Teil des Reaktionsgemisches abgeleitet und gegebenenfalls nach Abkühlung dem Reaktor wieder zugeführt wird. Ferner sind an den Schlaufenreaktoren Zuleitungen für die Ausgangsstoffe bzw. bereits teilweise umgesetzte Reaktionslösung und eine Ableitung für das Reaktionsgut angebracht. Die Ausgangsstoffe können dem Reaktor auf die Weise zugegeben werden, daß man sie zunächst vermischt und danach die Mischung in den Reaktor einspeist. Es kann aber auch so verfahren werden, daß man die Ausgangsstoffe an verschiedenen Orten in den Schlaufenreaktor einführt. Zweckmäßigerweise erfolgt die Zuführung der Ausgangsstoffe unter guter Vermischung mit dem Reaktionsgut nach üblichen Methoden, die dem Fachmann bekannt oder leicht zugänglich sind. In den Umlaufreaktoren beträgt das Gewichtsverhältnis der pro Zeiteinheit zugeführten Stoffe zu dem pro Zeiteinheit im Kreis geführten Reaktionsgemisch mindestens 1:5, vorzugsweise 1:10, bis 1:60.

Das den ersten Umlaufreaktor verlassende Reaktionsgemisch enthält, bezogen auf die Summe der eingesetzten Stoffe, mindestens 50 Gewichtsprozent, insbesondere 60—80 Gewichtsprozent an 3-Cyclohexen-1-carboxaldehyd. Zur Komplettierung der Reaktion wird dann das die Umlaufapparatur verlassende Reaktionsgemisch einem Nachreaktor zugeführt, in dem kein Reaktionsgemisch im Kreis geführt wird.

Erfindungsgemäß besonders vorteilhaft ist, das den Umlaufreaktor verlassende Reaktionsgut zunächst einem zweiten Umlaufreaktor, der unter bereits erwähnten Bedingungen betrieben wird, zuzuführen und erst dann das den zweiten Umlaufreaktor verlassende Reaktionsgemisch, das, bezogen auf die Summe der eingesetzten Rohstoffe einen Gehalt an 3-Cyclohexen-1-carboxaldehyd von mindestens 75, vorzugsweise 85—95 Gewichtsprozent aufweist, dem Nachreaktor zur Vervollständigung der Umsetzung zuführt.

Als Nachreaktor wird vorzugsweise ein Rohrreaktor verwendet, der im wesentlichen aus einem oder mehreren gebündelten Rohren besteht. In einer vorteilhaften Ausführungsform des erfindungsgemäßen Verfahrens wird durch das Verhältnis von den Nachreaktor durchströmendem Reaktionsgemisch zum Reaktorquerschnitt dafür gesorgt, daß eine Rückvermischung weitestgehend unterdrückt wird. In dem Nachreaktor wird die Umsetzung bei einer Temperatur von 100—240°C, vorzugsweise 140—170°C, durchgeführt. Das den Nachreaktor verlassende Reaktionsgemisch weist neben einem Gehalt von 90—93% 3-Cyclohexen-1-carboxaldehyd ca. 1,5% Wasser, nur ca. 2% Dimere und 2% Hochsieder und Polymere neben nicht umgesetztem Ausgangsmaterial auf.

Der durch das erfindungsgemäße Verfahren erhaltene 3-Cyclohexen-1-carboxaldehyd ist ein wertvolles Zwischenprodukt, z.B. zur Herstellung von Epoxidharzen, Riechstoffen und pharmazeutischen Wirkstoffen.

### Beispiel 1

Die Umsetzung wird in einer nachfolgend beschriebenen Laboranlage durchgeführt. Einem Umlaufreaktor von 2,9 l Inhalt wird ein Teil des Reaktionsgemisches am oberen Ende des Reaktors entnommen und nach dem Durchlaufen einer thermostatisierten Strecke über eine Pumpe dem Reaktor am unteren Ende wieder zugeführt. Acrolein und Butadien werden vor dem Eintritt in den Reaktor gemischt und oben am Reaktor dem Reaktionsgemisch zugeführt. Ein dem Zulauf entsprechender Teil des Reaktionsgemisches wird dem Reaktor entnommen und dem nachgeschalteten Rohrreaktor zugeführt, der bei einer Länge von 10 m ein Volumen von 1,1 l besitzt. Das den Rohrreaktor verlassende Reaktionsgemisch wird auf Normaldruck entspannt und destillativ aufgearbeitet. Zur Durchführung der Reaktion werden dem Umlaufreaktor stündlich 317 g (96 proz.) Acrolein, das 0,3% Hydrochinon enthält und 307 g Butadien (99 proz.) (Molverhältnis 1:1,04) zugeführt. Die mittlere Gesamtverweilzeit beträgt ca. 5,5 h. Die Temperatur im Schlaufenreaktor beträgt 130°C, im Nachreaktor 150°C; der Druck wird auf 25 bar gehalten.

Das den Umlaufreaktor verlassende Reaktionsgemisch enthält 77,5 Gewichtsprozent 3-Cyclohexen-1-carboxaldehyd; das den Nachreaktor verlassende Reaktionsgemisch weist einen Gehalt von 92,5% 3-Cyclohexen-1-carboxaldehyd auf, was 95,9% Ausbeute bei einem Acroleinumsatz von 96,9% bedeutet. Der Gehalt an Polymeren beträgt lediglich 2,3%.

Das Verhältnis von zugeführtem Reaktionsgemisch zu umgewälztem Reaktionsgemisch belief sich auf ca. 1:25.

### Beispiel 2

Wie vor, jedoch unter Zwischenschaltung eines weiteren Umlaufreaktors vom Volumen 2,8 l vor den Nachreaktor speist man stündlich 278 g Acrolein, stabilisiert mit 0,3% Hydrochinon und 296 g Butadien ein (Molverhältnis 1:1,13). Die Temperaturen der beiden Umlaufreaktoren betragen 120°C, des Nachreaktors 150°C; der Druck wird auf 25 bar gehalten. Der Gehalt an 3-Cyclohexen-1-carboxaldehyd beträgt im Reaktionsgemisch nach dem ersten Umlaufreaktor 77,6 Gewichtsprozent, nach dem zweiten Umlaufreaktor 89,4% und hinter dem Nachreaktor 93,6%. Bei einem Acroleinumsatz von 97,5% erhält man nach dem Destillieren bei 15 mmHg/62°C 93,7% Ausbeute an 3-Cyclohexen-1-carboxaldehyd. Der Rückstand beträgt weniger als 2%, bezogen auf die Summe der eingesetzten Stoffe.

In beiden Umlaufreaktoren beträgt das Verhältnis von zugeführtem Reaktonsgemisch zu umgewälztem Reaktionsgemisch ca. 1:25 bis 1:30.

### Beispiel 3

Wie vorstehend speist man pro Stunde 317 g Acrolein, stabilisiert mit 0,3% Hydrochinon, und 300 g Butadien (Molverhältnis 1:1,01) ein. Die Temperaturen betragen in Schlaufe 1 120°C, in Schlaufe 2 120°C und im Nachreaktor 150°C.

Der Gehalt an 3-Cyclohexen-1-carboxaldehyd beträgt im Reactionsgemisch vor Schlaufe 1 76,9%, nach Schlaufe 2 86,4% und nach dem Rohrreaktor 92,6%.

Der Acroleinumsatz beträgt 98,4%, die Ausbeute nach Destillation 92,4% und der Restgehalt an Polymeren 1,9%.

### Beispiel 4 (Gegenbeispiel)

In einem Rohrreaktor von 3,9 m Länge und einem Volumen von 250 ml speist man bei 130°C stündlich 212 g eines äquimolaren Gemisches aus mit 0,3% Hydrochinon stabilisieretem Acrolein und Butadien ein.

Nach ca. 12 h war der Rohrreaktor durch unlösliche Polymerablagerungen verstopft, so daß der Versuch abgebrochen werden mußte.

### Patentansprüche

1. Verfahren zur Herstellung von 3-Cyclohexen-1-carboxaldehyd durch Umsetzung von Acrolein mit Butadien bei erhöhter Temperatur und erhöhtem Druck, dadurch gekennzeichnet, daß man die Umsetzung kontinuierlich auf die Weise durchführt, daß zunächst in einem Umlaufreaktor, dem Acrolein und Butadien kontinuierlich zugeführt werden, bei Temperaturen zwischen 80°C und 160°C, vorzugsweise zwischen 100°C und 130°C gearbeitet wird, wobei man einen Teil des Reaktionsgemisches im Kreis führt, in dem das Gewichtsverhältnis der pro Zeiteinheit zugeführten Ausgangsstoffe zu dem pro Zeiteinheit umgewälzten Reaktionsgemisches 1:5 bis 1:60 beträgt, und man anschließend das den Umlaufreaktor verlassende Reaktionsgemisch, das mindestens 50 Gewichtsprozent an 3-Cyclohexen-1-carboxaldehyd enthält, einem weiteren Umlaufreaktor zuführt, in dem ein Teil des Reaktionsgemisches im Kreis geführt wird, wobei das Gewichtsverhältnis der pro Zeiteinheit vom ersten Umlaufreaktor zugeführten Reaktionslösung zu der pro Zeiteinheit umgewälzten Reaktionslösung mindestens 1:5 beträgt, und in dem die Reaktion bei einer Temperatur zwischen 100°C und 200°C, vorzugsweise zwischen 110°C und 150°C durchgeführt wird, und die den zweiten Umlaufreaktor verlassende Reaktionslösung, die einen Gehalt von mindestens 75% an 3-Cyclohexen-1-carboxalde- hyd aufweist, einem Nachreaktor zuführt, in dem kein Umlauf des Reaktionsgemisches erfolgt, und in dem die Reaktion bei 100 bis 240°C, vorzugsweise 140 bis 170°C vervollständigt wird und man anschließend das erhaltene Reaktionsgemisch durch fraktionierende Destillation trennt.

2. Verfahren nach Anspruch 1, dadurch gekennzeichnet, daß man das den ersten Umlaufreaktor verlassende Reaktionsgemisch unmittelbar einem Nachreaktor, in dem keine Rückvermischung stattfindet, zuführt und die Reaktion bei 100 bis 240°C, vorzugsweise 140 bis 170°C vervollständigt.

3. Verfahren nach den Ansprüchen 1 und 2, dadurch gekennzeichnet, daß man Acrolein mit einem Wassergehalt von maximal 7 Gewichtsprozent einsetzt.

4. Verfahren nach den Ansprüchen 1 bis 3, dadurch gekennzeichnet, daß das Molverhältnis zwischen eingesetztem Acrolein und Butadien 0,5:1 bis 2:1, vorzugsweise 0,9:1 bis 1,1:1 beträgt.

5. Verfahren nach den Ansprüchen 1 bis 4, dadurch gekennzeichnet, daß man dem Acrolein Polymerisationsinhibitoren zusetzt.

### Revendications

1. Procédé pour la préparation du 3-cyclohexène-1-carboxaldéhyde par réaction de l'acroléine avec le butadiène à haute température et sous pression élevée, caractérisé en ce que l'on effectue la réaction en continu en opérant d'abord à des températures comprises entre 80°C et 160°C, de préférence entre 100°C et 130°C, dans un réacteur à circulation auquel sont envoyés en continu l'acroléine et le butadiène, en mettant en circuit une partie du mélange réactionnel, dans lequel procédé le rapport pondéral des produits de départ introduits par unité de temps au mélange réactionnel mis en circuit par unité de temps étant de 1:5 à 1:60, et en envoyant ensuite le mélange réactionnel, quittant le réacteur à circulation, qui contient au moins 50% en poids de 3-cyclohexène-1-carboxaldéhyde, à un second réacteur à circulation dans lequel une partie du mélange réactionnel est mise en circuit, le rapport pondéral de la solution réactionnelle envoyée par unité de temps à partir du premier réacteur à circulation, à la solution réactionnelle mise en circuit par unité de temps étant d'au moins 1:5, et dans lequel on effectue la réaction à une température comprise entre 100°C et 200°C, de préférence entre 110°C et 150°C, et on envoie la solution réactionnelle, quittant le second réacteur à circulation, qui présente une teneur d'au moins 75% en 3-cyclohexène-1-carboxaldéhyde, à un post-réacteur dans lequel n'a lieu aucune circulation du mélange réactionnel, et dans lequel on achève la réaction à 100—240°C, de préférence à 140—170°C et on fonctionne ensuite par distillation fractionnée le mélange réactionnel obtenu.

2. Procédé selon la revendication 1, caractérisé en ce que le mélange réactionnel quittant le premier réacteur à circulation est envoyé directement à un post-réacteur dans lequel n'a lieu aucun mélangeage en retour, et la réaction est achevée à 100—240°C, de préférence à 140—170°C.

3. Procédé selon la revendication 1 ou 2, caractérisé en ce que l'on utilise de l'acroléine ayant une teneur en eau de 7% en poids au maximum.

4. Procédé selon l'une des revendications 1 à 3, caractérisé en ce que le rapport molaire entre acroléine et butadiène de départ est de 0,5:1 à 2:1, de préférence de 0,9:1 à 1,1:1.

5. Procédé selon l'une des revendications 1 à 4, caractérisé en ce que l'on ajoute des inhibiteurs de polymérisation à l'acroléine.

**Claims**

1. A process for the production of 3-cyclohexene-1-carboxyaldehyde by reaction of acrolein with butadiene at elevated temperature and elevated pressure, characterized in that the reaction is carried out continuously by initially working in a circulation reactor, into which acrolein and butadiene are continuously introduced, at temperatures in the range from 80 to 160°C and preferably at temperatures in the range from 100 to 130°C, part of the reaction mixture, in which the ratio by weight of the starting materials introduced per unit of time to the reaction mixture circulated per unit of time is from 1:5 to 1:60, being circulated, and subsequently delivering the reaction mixture leaving the circulation reactor, which contains at least 50% by weight 3-cyclohexene-1-carboxaldehyde, to another circulation reactor in which the reaction mixture is partly circulated, the ratio by weight of the reaction solution fed to the first circulation reactor per unit of time to the reaction solution circulated per unit of time being at least 1:5, and in which the reaction is carried out at a temperature in the range from 100 to 200°C and preferably at a temperature in the range from 110 to 150°C, and delivering the reaction solution leaving the second circulation reactor, which contains at least 75% 3-cyclohexene-1-carboxaldehyde, to an after-reactor in which the reaction mixture is not circulated and in which the reaction is completed at 100 to 240°C and preferably at 140 to 170°C and subsequently separating the reaction mixture obtained by fractional distillation.

2. A process as claimed in claim 1, characterized in that the reaction mixture leaving the first circulation reactor is delivered immediately to an after-reactor, in which no back-mixing occurs, and the reaction is completed at 100 to 240°C and preferably at 140 to 170°C.

3. A process as claimed in claims 1 and 2, characterized in that acrolein having a water content of at most 7% by weight is used.

4. A process as claimed in claims 1 to 3, characterized in that the molar ratio between the acrolein and the butadiene is from 0.5:1 to 2:1 and preferably from 0.9:1 to 1.1:1.

5. A process as claimed in claims 1 to 4, characterized in that polymerization inhibitors are added to the acrolein.